(19) Europäisches Patentamt

European Patent Office

Office européen des brevets

(11) Veröffentlichungsnummer: **0 280 135 B1**

(12) **EUROPÄISCHE PATENTSCHRIFT**

(45) Veröffentlichungstag der Patentschrift: 25.09.91

(51) Int. Cl.⁵: **A61K 37/02, A61K 49/02**

(21) Anmeldenummer: **88102143.0**

(22) Anmeldetag: **13.02.88**

(54) Protein C-Inhibitor (PCI) als Pharmazeutikum und Diagnostikum, Verfahren zu Herstellung eines solchen Pharmazeutikums oder Diagnostikums sowie ein Mittel enthaltend PCI.

(30) Priorität: 23.02.87 DE 3705745

(43) Veröffentlichungstag der Anmeldung:
31.08.88 Patentblatt 88/35

(45) Bekanntmachung des Hinweises auf die
Patenterteilung:
25.09.91 Patentblatt 91/39

(84) Benannte Vertragsstaaten:
AT BE CH DE ES FR GB IT LI LU NL SE

(56) Entgegenhaltungen:

CHEMICAL ABSTRACTS, Band 98, Nr. 5, 31.
Januar 1983, Seite 312, Zusammenfassung
Nr. 30383t, Columbus, Ohio, US; K. SUZUKI et
al.: "Protein C inhibitor. Purification from human plasma and characterization", & J.
BIOL. CHEM. 1983, 258(1), 163-8

CHEMICAL ABSTRACTS, Band 101, Nr. 17, 13.
August 1984, Seite 410, Zusammenfassung
Nr. 52463b, Columbus, Ohio, US; K. SUZUKI:
"Regulation of factor V-associated blood
coagulation by protein C and its inhibitor", &
MIE MED. J. 1984, 33 (3), 297-321

(73) Patentinhaber: BEHRINGWERKE Aktiengesellschaft
Postfach 1140
W-3550 Marburg 1(DE)

(72) Erfinder: Stief, Thomas, Dr.
Friedrichstrasse 40
W-3550 Marburg(DE)
Erfinder: Heimburger, Norbert, Prof. Dr.
Sonnenhang 10
W-3550 Marburg(DE)
Erfinder: Radtke, Klaus-Peter, Dr.
Sandweg 34
W-3550 Marburg(DE)

(74) Vertreter: Klein, Otto, Dr. et al
Hoechst AG Zentrale Patentabteilung Postfach 80 03 20
W-6230 Frankfurt am Main 80(DE)

## Beschreibung

Die Erfindung betrifft Protein C-Inhibitor (PCI) als Pharmazeutikum, das heißt als Therapeutikum oder in vivo-Diagnostikum, oder in vitro-Diagnostikum und besonders zur Diagnose und Therapie von tumorösen Erkrankungen, ein Verfahren zur Herstellung eines solchen Pharmazeutikums oder Diagnostikums sowie eine Zusammensetzung enthaltend Protein C-Inhibitor.

Es ist bekannt, daß Plasminogen-Aktivatoren (PA) beim Tumorwachstum und bei der Tumormetastasierung eine Rolle spielen. Bei Tumorpatienten treten häufig Hämorrhagien auf, die mit einem Konzentrationsanstieg von Plasminogen-aktivatoren im Blut in Zusammenhang gebracht werden. Injektionen von Antikörpern gegen Urokinase, einem Plasminogen-Aktivator, führten bei Mäusen mit Melanomen zu einer signifikant niedrigeren Metastasierungs- und einer erhöhten Überlebensrate.

Bei malignen Tumoren ist eine Ermittlung des Tumorstadiums vor Behandlungsbeginn für die Art der Behandlung und die klinische Prognose von großer Bedeutung. In vielen Fällen gibt eine herkömmliche, nichtinvasive Diagnostik nicht genügend Aufschluß über das Tumorstadium. Bei Verfahren nach dem Stand der Technik werden Tumorinfiltration und Tumormetastatisierung mit Hilfe radioaktiv markierter monoklonaler oder polyklonaler Antikörper gegen auf den Tumorzellen vorkommende Antigene verfolgt. Es ist auch bekannt, daß Zellgifte, die an Antikörper gegen Tumorzellen gebunden sind, in vitro zytotoxisch auf Tumorzellen wirken können. Die Nachteile dieser Methoden nach dem Stand der Technik liegen zum einen darin, daß körperfremdes Material verwendet wird und damit eine Immunantwort induziert werden kann, die zu Immunkomplexkrankheiten führen kann. Zum anderen wirken an Antiköper gebundene Zellgifte auch auf andere nichtentartete Körperzellen in unspezifischer Weise zytotoxisch.

Es wurde nun überraschenderweise gefunden, daß Protein C-Inhibitor (PCI) gegebenenfalls in Anwesenheit von sulfatierten Sacchariden inaktive Komplexe mit Plasminogen-Aktivatoren (PA) bildet. In Anwesenheit von sulfatierten Sacchariden werden Komplexe bevorzugt mit Urokinase (UK) gebildet. Die Geschwindigkeit der Reaktion von Plaminogen-Aktivatoren und Protein C-Inhibitor wird durch Zugabe eines sulfatierten Saccharids wie Heparin drastisch gesteigert.

Suzuki et al. (J.Biol.Chem 1983, 258 (1), 163-8) und Suzuki (Mie Med.J. 1984, 33(3), 297-321) beschreiben die Isolierung von Protein C-Inhibitor und seine Wirkung auf die Faktor V abhängige Gerinnung.

Aufgrund der Komplexbildung von Protein C-Inhibitor mit Plasminogen-Aktivatoren und der dadurch bewirkten Inhibierung diese Aktivatoren, die beim Wachstum von Tumoren und ihrer Metastasierung eine Rolle spielen, ist dieser Inhibitor zur Behandlung von Tumoren geeignet.

Kennzeichen vieler solider neoplastischer Erkrankungen ist eine unkontrollierte Angiogenese. Unter anderem kann eine solche Angiogenese durch Urokinase, die von Zellen freigesetzt wird, induziert werden. Die Inhibition dieser Urokinase durch Protein C-Inhibitor führt daher auch zu einem Sistieren von Krankheiten nichtneoplastischer Art.

Persistierende Angiogenese stellt die Pathogeneseform vieler nichtneoplastischer Erkrankungen dar. Diabetische Retinopathie, retrolentale Fibroplasie, neovasculäre Glaucome, rheumatische Arthritis, Hemangiome, Angiofibrome, Psoriasis und kapilläre Proliferation innerhalb atherosclerotischer Plaques sind Beispiele für sogenannte "angiogene Erkrankungen".

Gegenstand der Erfindung ist deshalb Protein C-Inhibitor als Pharmazeutikum, vorzugsweise zur in vivo-Diagnose oder Behandlung von angiogenen Erkrankungen und besonders von Tumoren.

Da Protein C-Inhibitor in humanem Blut in einer Konzentration von 2 - 5 µg/ml vorkommt, sind Nebenwirkungen, wie sie bei Anwendung von artfremden Proteinen, beispielsweise solchen Antikörpern zu erwarten sind, nicht zu befürchten. Weiterhin ist die Ankopplung eines Zellgiftes entbehrlich, da der Inhibitor selbst eine plasminogenaktivatorneutralisierende und damit antimetastasierende Wirkung hat.

Für diagnostische Zwecke kann der Inhibitor mit einem Markierungsmittel, vorzugsweise einem fluoreszierenden, lumineszierenden oder radioaktiven Mittel oder einem Enzym, bevorzugt einem radioaktiven Isotop oder einem Enzym versehen sein.

Der Inhibitor kann zur in vitro- wie zur in vivo-Diagnose verwendet werden.

Gegenstand der Erfindung ist auch eine Zusammensetzung enthaltend Protein C-Inhibitor und einen Träger.

Geeignete Trägermaterialien sind solche, die für pharmazeutische, also therapeutische oder in vivo-diagnostische, oder in vitro-diagnostische Zwecke geeignet sind.

Eine solche Zusammensetzung kann, gegebenenfalls markierten, Protein C-Inaktivator in einer Konzentration von 0,001 bis 1 000, vorzugsweise 0,01 bis 200, besonders bevorzugt 0,1 bis 10 mg/ml und gegebenenfalls ein sulfatiertes Saccharid, beispielsweise Heparin, Heparinsulfat, Pentosansulfat, Dextransulfat, Keratansulfat, Chondroitinsulfat oder Dermatansulfat, vorzugsweise Heparin, Dextran- oder Pentosansulfat, in einem molaren Verhältnis von 10 : 1 bis 1 : 100 000, vorzugsweise 10 : 1 bis 1 : 10 000 und gegebenenfalls stabilisierende Zusätze in einer Konzentration von 0,1 bis 2, bevorzugt 0,5 bis 1 g/100 ml, beispielsweise Albumin enthalten.

Als Markierungen kommen die für diesen Zweck bekannten in Frage, besonders eine solche durch radioaktive Isotope, beispielsweise mit $^{111}$In.

Gegenstand der Erfindung ist auch ein Verfahren zur Herstellung eines Mittels zur Diagnose von angiogenen Erkrankungen oder Tumoren, dadurch gekennzeichnet, daß Protein C-Inhibitor mit einem Markierungsmittel versehen und der markierte Inhibitor gegebenenfalls mit einem sulfatierten Saccharid und gegebenenfalls mit einem Stabilisator versetzt wird.

Eine radioaktive Markierung, beispielsweise mit $^{111}$In ist entsprechend der Methode, die in Biochem. Biophys. Res. Commun. 77, 581-585, 1977 beschrieben ist, möglich.

Gegenstand der Erfindung ist weiterhin ein Mittel zur Behandlung von angiogenen Erkrankungen oder Tumoren, das Protein C-Inhibitor und gegenenfalls ein sulfatiertes Saccharid in einem flüssigen Träger enthält.

Bevorzugt enthält eine solche Zusammensetzung Protein C-Inhibitor in einer Konzentration von $10^{-4}$ bis $10^{3}$, vorzugsweise von 0,01 bis 200 und besonders bevorzugt 0,1 bis 10 mg/ml.

Besonders geeignet ist ein Mittel, das Protein C-Inhibitor und ein sulfatiertes Saccharid, beispielsweise Heparin, Heparinsulfat, Pentosansulfat, Galaktosansulfat, Dextransulfat, Keratansulfat, Chondroitin oder Dermatansulfat, vorzugsweise Heparin, Dextran- oder Pentosansulfat, in einem molaren Verhältnis (PCI/sulfatiertes Saccharid) von 10 : 1 bis 1 : 100 000, bevorzugt von 10 : 1 bis 1 : 10 000, besonders bevorzugt 1 : 20 enthält.

Gegenstand der Erfindung ist auch ein Verfahren zur Herstellung eines Mittels zur Behandlung von angiogenen Erkrankungen oder Tumoren, dadurch gekennzeichnet, daß Protein C-Inhibitor, gegebenenfalls ein sulfatiertes Saccharid und ein pharmazeutisch unbedenklicher Träger und/oder ein Stabilisator und/oder ein Hilfsstoff zu einem zur Therapie geeigneten Mittel verarbeitet werden.

Ein solches sulfatiertes Saccharid ist vorzugsweise Heparin, Dextran- oder Pentosansulfat, besonders bevorzugt Pentosansulfat, das ein mittleres Molekulargewicht von 3 -15 000, einen Sulfatierungsgrad von ca. 20 % besitzt und wird in einem molaren Verhältnis (PCI/sulfatiertes Saccharid) von 10 : 1 bis 1 : 100 000, vorzugsweise von 10 : 1 bis 1 : 10 000, besonders bevorzugt 1 : 10 bis 1 : 100 zugesetzt.

Gegebenenfalls wird das Mittel mit Stabilisatoren wie Albumin versetzt, sterilfiltriert und lyophilisiert.

Die Erfindung soll durch folgende Beispiele erläutert werden:

Beispiel 1:

500 µl einer Lösung von PCI (20 µg/ml) werden mit 500 µl einer Lösung von UK in 50 mmol/l Tris-Puffer (10 µg/ml), 0,1 mol/l NaCl, pH 8,5, in Gegenwart und Abwesenheit von 5 Einheiten Heparin/ml bei Raumtemperatur inkubiert. Nach definierten Zeiten werden dem Inkubationsansatz 100 µl Proben entnommen und in einer Elektrophorese auf einem Natrium-dodecylsulfat enthaltenden Polyacrylamid-Gel ("SDS-PAA-Gelelektrophorese") (8 g/100 ml Polyacrylamid) aufgetrennt. Nach einem anschliessenden Westernblot (Trends in Biochemical Science, 103-106, 1985) werden die dabei erhaltenen Cellulosefolien entweder mit Antikörpern gegen UK oder PCI inkubiert, mit Puffer gewaschen und gefärbt. In beiden Fällen tritt zusätzlich zur Bande des entsprechenden Proteins nach 30' ohne Heparin, nach 2' mit Heparin eine Bande mit einem Molekulargewicht Mw = 110 000 auf, die dem Komplex aus UK und PCI entspricht.

Beispiel 2:

In den in Beispiel 1 genannten Proben wurde die Aktivität der eingesetzten UK bestimmt, indem zu der jeweiligen Probe 1000 µl Substratlösung S 2444 $^{(R)}$Kabi Vitrum, 0,3 mmol/l, in 50 mmol/l Tris, 0,1 M NaCl, pH 8,5, pipettiert wurden. Die Umsetzung des Substrates wurde nach einer Inkubation von 30 Minuten bei 37°C durch Zugabe von 100 µl 8,5 mol/l Essigsäure abgestoppt und die durch die Abspaltung des chromogenen Restes para-Nitroanilin entstandene Extinktionsdifferenz in einem Photometer gemessen. Anhand einer unter den genannten Bedingungen mit UK erstellten Bezugskurve wurden die UK-Konzentrationen der Proben ermittelt. Auf diese Weise konnten im Vergleich zum Ausgangswert durch eine mitgeführte Kontrolle, die nur UK enthielt, eine Inaktivierung von UK, abhängig von der PCI-Konzentration, gezeigt werden. Dabei zeigte sich, daß die Inaktivierung durch Heparin ca. 20fach beschleunigt wurde.

**Patentansprüche**
**Patentansprüche für folgende Vertragsstaaten : AT, BE, CH, DE, FR, GB, IT, LI, LU, NL, SE**

1. Protein C-Inhibitor zur Verwendung als Arzneimittel.

2. Protein C-Inhibitor gemäß Anspruch 1 zur Behandlung angiogener Erkrankungen.

3. Protein C-Inhibitor gemäß Anspruch 1 zur Behandlung von Tumoren.

**4.** Protein C-Inhibitor zur Verwendung als Diagnostikum.

**5.** Protein C-Inhibitor gemäß Anspruch 4 zur Diagnose angiogener Erkrankungen.

**6.** Protein C-Inhibitor gemäß Anspruch 4 zur Diagnose von Tumoren.

**7.** Zusammensetzung enthaltend Protein C-Inhibitor und einen Träger und gegebenenfalls ein sulfatiertes Saccharid.

**8.** Verfahren zur Herstellung eines Mittels nach Anspruch 7 zur Behandlung von angiogenen Erkrankungen oder Tumoren, dadurch gekennzeichnet, daß Protein C-Inhibitor, gegebenenfalls ein sulfatiertes Saccharid und ein pharmazeutisch unbedenklicher Täger und/oder ein Stabilisator und/oder ein Hilfsstoff zu einem zur Therapie geeigneten Mittel verarbeitet werden.

**9.** Verfahren zur Herstellung eines Mittels nach Anspruch 7 zur Diagnose von angiogenen Erkrankungen oder Tumoren, dadurch gekennzeichnet, daß Protein C-Inhibitor mit einem Markierungsmittel versehen und der markierte Inhibitor gegebenenfalls mit einem sulfatierten Saccharid und gegebenenfalls mit einem Stabilisator versetzt wird.

**10.** Verwendung von Protein C-Inhibitor zur Herstellung eines Mittels nach Anspruch 7 zur Behandlung von angiogenen Erkrankungen oder Tumoren.

**11.** Verwendung von Protein C-Inhibitor zur Herstellung eines Mittels nach Anspruch 7 zur Diagnose von angiogenen Erkrankungen oder Tumoren.

**Patentansprüche für folgenden Vertragsstaat : ES**

**1.** Verfahren zur Herstellung eines Mittels zur Behandlung von angiogenen Erkrankungen oder Tumoren, dadurch gekennzeichnet, daß Protein C-Inhibitor, gegebenenfalls ein sulfatiertes Saccharid und ein pharmazeutisch unbedenklicher Träger und/oder ein Stabilisator und/oder ein Hilfsstoff zu einem zur Therapie geeigneten Mittel verarbeitet werden.

**2.** Verfahren zur Herstellung eines Mittels zur Diagnose von angiogenen Erkrankungen oder Tumoren, dadurch gekennzeichnet, daß Protein C-Inhibitor mit einem Markierungsmittel versehen und der markierte Inhibitor gegebenenfalls mit einem sulfatierten Saccharid gegebenenfalls mit einem Stabilisator versetzt wird.

**Claims**
**Claims for the following Contracting States : AT, BE, CH, DE, FR, GB, IT, LI, LU, NL, SE**

**1.** Protein C inhibitor for use as medicament.

**2.** Protein C inhibitor as claimed in claim 1 for the treatment of angiogenic diseases.

**3.** Protein C inhibitor as claimed in claim 1 for the treatment of tumors.

**4.** Protein C inhibitor for use as diagnostic agent.

**5.** Protein C inhibitor as claimed in claim 4 for the diagnosis of angiogenic diseases.

**6.** Protein C inhibitor as claimed in claim 4 for the diagnosis of tumors.

**7.** A composition containing protein C inhibitor and a vehicle and, where appropriate, a sulfated saccharide.

**8.** A process for the preparation of an agent as claimed in claim 7 for the treatment of angiogenic diseases or tumors, which comprises processing protein C inhibitor, where appropriate a sulfated saccharide and a pharmaceutically acceptable vehicle and/or a stabilizer and/or an auxiliary to give an agent suitable for therapy.

**9.** A process for the preparation of an agent as claimed in claim 7 for the diagnosis of angiogenic diseases or tumors, which comprises providing protein C inhibitor with a labeling agent, and mixing the labeled inhibitor, where appropriate, with a sulfated saccharide and, where appropriate, with a stabilizer.

**10.** The use of protein C inhibitor for the preparation of an agent as claimed in claim 7 for the treatment of angiogenic diseases or tumors.

**11.** The use of protein C inhibitor for the preparation of an agent as claimed in claim 7 for the diagnosis of angiogenic diseases or tumors.

**Claims for the following Contracting States : ES**

**1.** A process for the preparation of an agent for the treatment of angiogenic diseases or tumors, which comprises processing protein C inhibitor, where appropriate a sulfated saccharide and a pharmaceutically acceptable vehicle and/or a stabilizer and/or an auxiliary to give an agent suitable for therapy.

2. A process for the preparation of an agent for the diagnosis of angiogenic diseases or tumors, which comprises providing protein C inhibitor with a labeling agent, and mixing the labeled inhibitor, where appropriate, with a sulfated saccharide and, where appropriate, with a stabilizer.

## Revendications
### Revendications pour les Etats contractants suivants : AT, BE, DE, CH, FR, GB, IT, LI, LU, NL, SE

1. Inhibiteur-protéine C destiné à être utilisé en tant que médicament.

2. Inhibiteur-protéine C selon la revendication 1 pour le traitement de maladies angiogènes.

3. Inhibiteur-protéine C selon la revendication 1 pour le traitement de tumeurs.

4. Inhibiteur-protéine C à utiliser en tant qu'agent de diagnostic.

5. Inhibiteur-protéine C selon la revendication 4 pour le diagnostic de maladies angiogènes.

6. Inhibiteur-protéine C selon la revendication 4 pour le diagnostic de tumeurs.

7. Composition contenant l'inhibiteur-protéine C et un véhicule et, le cas échéant, un saccharide sulfaté.

8. Procédé pour préparer un agent selon la revendication 7 pour le traitement de maladies angiogènes ou de tumeurs, caractérisé en ce que l'on transforme l'inhibiteur-protéine C, le cas échéant un saccharide sulfaté et un véhicule pharmaceutiquement acceptable et/ou un agent stabilisant et/ou un adjuvant, en un agent adapté à la thérapeutique.

9. Procédé pour préparer un agent selon la revendication 7 pour le diagnostic de maladies angiogènes ou de tumeurs, caractérisé en ce que l'on munit l'inhibiteur-protéine C d'un agent de marquage et l'on utilise l'inhibiteur marqué, le cas échéant avec un saccharide sulfaté, et, le cas échéant, avec un agent stabilisant.

10. Utilisation de l'inhibiteur-protéine C pour préparer un agent selon la revendication 7 pour le traitement de maladies angiogènes ou de tumeurs.

11. Utilisation de l'inhibiteur-protéine C pour préparer un agent selon la revendication 7 pour le diagnostic de maladies angiogènes ou de tumeurs.

### Revendications pour l'Etat contractant suivant : ES

1. Procédé pour préparer un agent pour le traitement de maladies angiogènes ou de tumeurs, caractérisé en ce que l'on transforme l'inhibiteur-protéine C, le cas échéant un saccharide sulfaté et un véhicule pharmaceutiquement acceptable et/ou un agent stabilisant et/ou un adjuvant, en un agent adapté à la thérapeutique.

2. Procédé pour préparer un agent pour le diagnostic de maladies angiogènes ou de tumeurs, caractérisé en ce que l'on munit l'inhibiteur-protéine C d'un agent de marquage et l'on utilise l'inhibiteur marqué, le cas échéant avec un saccharide sulfaté, le cas échéant avec un agent stabilisant.